(19) Europäisches Patentamt / European Patent Office / Office européen des brevets

(11) **EP 2 152 335 B1**

(12) **EUROPÄISCHE PATENTSCHRIFT**

(45) Veröffentlichungstag und Bekanntmachung des Hinweises auf die Patenterteilung:
**11.01.2012 Patentblatt 2012/02**

(21) Anmeldenummer: 08758922.2

(22) Anmeldetag: **31.05.2008**

(51) Int Cl.:
**A61M 1/30** *(2006.01)*

(86) Internationale Anmeldenummer:
**PCT/EP2008/004353**

(87) Internationale Veröffentlichungsnummer:
**WO 2008/148505 (11.12.2008 Gazette 2008/50)**

(54) **Vorrichtung zur Blutbehandlung für den Einnadel-Betrieb**

Device for blood treatment in single-needle operating mode

Dispositif de traitement du sang en fonctionnement à une seule aiguille

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **04.06.2007 DE 102007026009**

(43) Veröffentlichungstag der Anmeldung:
**17.02.2010 Patentblatt 2010/07**

(73) Patentinhaber: **Fresenius Medical Care Deutschland GmbH
61352 Bad Homburg v.d.H. (DE)**

(72) Erfinder:
• **GÖTZ, Günther
61440 Oberursel (DE)**

• **HERRENBAUER, Michael
61267 Neu-Anspach (DE)**
• **LAUER, Martin
66606 St. Wendel (DE)**
• **MÜLLER, Ralf
61348 Bad Homburg (DE)**

(74) Vertreter: **Oppermann, Frank
Luderschmidt, Schüler & Partner
Patentanwälte
John-F-Kennedy-Strasse 4
65189 Wiesbaden (DE)**

(56) Entgegenhaltungen:
**EP-A- 0 405 094      EP-A- 0 472 480
DE-A1-102005 001 779      US-A- 4 655 742**

EP 2 152 335 B1

## Beschreibung

Die Erfindung bezieht sich auf eine Vorrichtung zur Blutbehandlung für den Einnadel-Betrieb, die über einen extrakorporalen Blutkreislauf verfügt, der eine zu dem Einlass einer Blutbehandlungseinheit führende Blutzuführleitung und eine von dem Auslass der Blutbehandlungseinheit abgehende Blutrückführleitung aufweist, wobei Blutzuführ- und -rückführleitung zu einer gemeinsamen Nadel (Kanüle) führen bzw. von einer gemeinsamen Nadel abgehen.

Blutbehandlungsvorrichtungen mit einer Blutbehandlungseinheit, die von dem Blut eines Patienten durchströmt wird, sind allgemein bekannt. Zu diesen zählen beispielsweise die bekannten Hämodialyse-, Hämofiltrations- oder Hämodiafiltrationsvorrichtungen. Die bekannten Blutbehandlungsvorrichtungen können im Einnadel- oder Zweinadel-Betrieb betrieben werden.

Bei der Zweinadel-Technik wird das Blut über eine erste Nadel von einem Blutgefäß des Patienten abgezogen, in die Blutbehandlungseinheit der Blutbehandlungsvorrichtung geleitet und über eine zweite Nadel in ein Blutgefäß des Patienten zurückgeführt. Für die Entnahme und Rückführung des Blutes finden auswechselbare Schlauchsysteme mit einer Blutzuführ- und -rückführleitung Verwendung, an denen die beiden Nadeln angeschlossen sind. Diese wegzuwerfenden Schlauchsysteme werden auch als Disposable bezeichnet.

Bei der Einnadel-Technik erfolgt die Entnahme und Rückführung des Blutes über eine einzige Nadel. Das dem Patienten entnommene Blut wird während einer arteriellen Phase in einem Reservoir gespeichert, um dann in einer venösen Phase aus dem Speicher in den Blutkreislauf des Patienten durch dieselbe Nadel zurückgeführt zu werden.

Eine Blutbehandlungsvorrichtung für den Einnadel-Betrieb ist aus der EP-A-0 472 480 B 1 bekannt. Bei einer Ausführungsform des bekannten Blutbehandlungsgeräts sind zwei Blutexpansionskammern zum temporären Speichern von Blut vorgesehen, die stromauf und stromab der Blutbehandlungseinheit angeordnet sind. Das Blutbehandlungsgerät weist eine Regeleinrichtung auf, die den Druck in den Expansionskammern im Wesentlichen konstant hält. Zur Detektion der Flüssigkeitspegel in den Expansionskammern sind Niveausensoren vorgesehen.

Die DE 10 2005 001 779 A1 beschreibt ein Set für ein Disposable zum Betreiben einer Blutbehandlungsvorrichtung im Einnadel- oder Zweinadel-Betrieb. Das Disposable umfasst neben der Blutzuführ- und -rückführleitung zum Anschluss an eine Blutbehandlungseinheit eine Expansionseinheit, die für den Einnadel-Betrieb zur Vergrößerung des Volumens an die Luftabscheideeinheit ankoppelbar ist. Während des Einnadel-Betriebs wird in der arteriellen Phase Blut durch die Blutzuführleitung in die Blutbehandlungseinheit und aus der Blutbehandlungseinheit in die Luftabscheide- und Expansionseinheit gefördert, wobei die Blutzufuhr zum Patienten unterbrochen ist. Dabei wird ein vorgegebener Druck in der Luftabscheide- und Expansionseinheit aufgebaut, der mit einer Druckmesseinheit überwacht wird. Mit einer Druckufteinheit kann durch Betätigung einer Luftpumpe, die zwischen einen Tank und die Expansionseinheit geschaltet ist, ein vorgegebener Druck in der Expansions- und Luftabscheideeinheit eingestellt werden. Darüber hinaus wird vorgeschlagen, mit Hilfe der Messewerte von drei Drucksensoren und der bekannten Systemvolumina das Blutvolumen in der Expansions- und Luftabscheideeinheit zu berechnen. Ferner wird vorgeschlagen, die Luftpumpe zur Regelung des Drucks in der venösen Phase zu verwenden, so dass die Förderrate des Blutes optimal angepasst werden kann.

Die EP 0 405 094 A2 beschreibt eine Vorrichtung zur Blutbehandlung im Einnadel-Betrieb, die über einen Plasmaseparator verfügt. Der Einnadel-Betrieb umfasst zwei Schritte. In dem ersten Schritt wird das Blut des Patienten in einem Blutsammelbeutel gesammelt. In dem zweiten Schritt werden das gesammelte Blut aus dem Blutsammelbeutel in den Plasmaseparator überführt, das Blutplasma aus dem Plasmaseparator in einen Plasmasammelbeutel überführt und die verbleibenden Blutkomponenten zu dem Patienten zurückgeführt. Der extrakorporale Blutkreislauf umfasst eine Blutzuführleitung und eine Blutrückführleitung, die zu einer gemeinsamen Nadel führen. Der Blut- und Plasmasammelbeutel befinden sich in Aufnahmeeinheiten, in denen die Beutel einem Über- bzw. Unterdruck ausgesetzt werden können.

Der Erfindung liegt die Aufgabe zu Grunde, eine Vorrichtung zur Blutbehandlung für den Einnadel-Betrieb zu schaffen, bei der das Blutvolumen im Blutreservoir ohne Niveausensoren ermittelt und das Verhalten im Fehlerfall verbessert werden kann.

Die Lösung dieser Aufgaben erfolgt erfindungsgemäß mit den Merkmalen des Patentanspruchs 1. Vorteilhafte Ausführungsformen der Erfindung sind Gegenstand der Unteransprüche.

Die erfindungsgemäße Vorrichtung zur Blutbehandlung verfügt über einen extrakorporalen Blutkreislauf, der eine zu dem Einlass einer Blutbehandlungseinheit führende Blutzuführleitung und eine von dem Auslass der Blutbehandlungseinheit abgehende Blutrückführleitung aufweist. Dieser extrakorporale Blutkreislauf mit der Blutbehandlungseinheit braucht nicht Teil der Blutbehandlungsvorrichtung zu sein, sondern kann ein zur einmaligen Verwendung bestimmtes Disposable sein, das für die Blutbehandlung in die Behandlungseinheit eingelegt wird.

Darüber hinaus weist die Blutbehandlungsvorrichtung Mittel zum Sammeln von Blut und Mittel zum Speichern von Gas, insbesondere Luft auf. Bei den Mitteln zum Sammeln von Blut und zum Speichern von Gas handelt es sich jeweils um ein Reservoir, das ein abgeschlossenes Volumen bildet, beispielsweise um eine Blut- bzw. Luftkammer mit einem vorgegebenen Volumen.

Die Mittel zum Sammeln von Blut stehen mit den Mitteln zum Speichern von Gas in Verbindung, so dass in der arteriellen Phase beim Befüllen der Mittel zum Sammeln von Blut aus den Mitteln zum Sammeln von Blut verdrängte Luft in die Mittel zum Speichern von Gas und in der venösen Phase Luft aus den Mitteln zum Speichern von Gas in die Mittel zum Sammeln von Blut gelangen kann, wodurch das zuvor in der arteriellen Phase gesammelte Blut aus den Mitteln zum Sammeln von Blut verdrängt wird. Dadurch wird erreicht, dass in der arteriellen Phase dem Patienten Blut entnommen und in der venösen Phase das mit der Blutbehandlungseinheit behandelte Blut dem Patienten wieder zurückgeführt wird.

Die Verbindung zwischen den Mitteln zum Sammeln von Blut und den Mitteln zum Speichern von Gas weist einen ersten und zweiten Verbindungspfad auf. Der erste Verbindungspfad verbindet die Mittel zum Sammeln von Blut und zum Speichern von Gas derart, dass in der arteriellen Phase aus den Mitteln zum Sammeln von Blut verdrängte Luft zu den Mitteln zum Speichern von Gas überführt wird, wobei in dem ersten Verbindungspfad Mittel zum Unterbrechen der Verbindung vorgesehen sind. Der zweite Verbindungspfad verbindet die Mittel zum Sammeln von Blut und Speichern von Gas derart, dass in der venösen Phase in den Mitteln zum Speichern von Gas gespeicherte Luft zu den Mitteln zum Sammeln von Blut überführt wird. Der zweite Verbindungspfad enthält Mittel zum Verdichten von Gas, so dass die in den Mitteln zum Speichern von Gas gespeicherte Luft in die Mittel zum Sammeln von Blut überführt werden kann. Mit den Mitteln zum Verdichten der Luft kann in den Mitteln zum Sammeln von Blut ein vorgegebener Druck sowohl während der arteriellen als auch der venösen Phase aufgebaut werden. Die Mittel zum Verdichten von Gas können beispielsweise als ein konventioneller Kompressor ausgebildet sein.

Die Mittel zum Sammeln von Blut und Speichern von Gas bilden zusammen mit dem ersten und zweiten Verbindungspfad ein abgeschlossenes Volumen, in das kein Gas eintreten bzw. aus dem kein Gas austreten kann. Nur zum Zwecke der Initialisierung des Systems wird das abgeschlossene Volumen be-/entlüftet. Der erste Verbindungspfad und der zweite Verbindungspfad können von getrennten Leitungen gebildet werden. Der erste und zweite Verbindungspfad können aber auch einen gemeinsamen Leitungsabschnitt umfassen.

Eine Luftinfusion in den Patienten kann dadurch mit erhöhter Sicherheit ausgeschlossen werden, dass in den Mitteln zum Speichern von Gas ein Druck eingestellt wird, der in keinem Betriebsfall größer als der Druck ist, der sich in den Mitteln zum Sammeln von Blut einstellt. Dadurch wird erreicht, dass Luft nur mit den Mitteln zum Verdichten von Gas von den Mitteln zum Speichern von Gas in die Mittel zum Sammeln von Blut gelangen kann. Bei einem Ausfall der Mittel zum Verdichten von Gas, beispielsweise einer Leckage des Kempressors, kann hingegen keine Luft in die Mittel zum Sammeln von Blut gelangen. Dadurch wird ausgeschlossen, dass die Mittel zum Sammeln von Blut unbeabsichtigt leer laufen, wodurch Luft in den Patienten gelangen könnte.

In der Praxis wird angestrebt, dass der Rückgabedruck, mit dem Blut aus den Mitteln zum Sammeln von Blut verdrängt wird, möglichst niedrig einstellbar ist, während das Schlagvolumen möglichst groß ist. Um die Bedingung, dass bei einem Ausfall des Kompressors nicht Luft aus den Mitteln zum Speichern von Gas in die Mittel zum Sammeln von Blut gelangen kann, bei niedrigem Rückgabedruck und großem Schlagvolumen erfüllen zu können, wird das System zur Initialisierung der aufeinander folgenden arteriellen und venösen Phasen beim Zeitpunkt der Umschaltung von der arteriellen auf die venöse Phase, d.h. zu dem Zeitpunkt des oberen Umschaltpunktes, bei dem die Mittel zum Sammeln von Blut befüllt sind, auf einen vorgegebenen Druck, vorzugsweise den Umgebungsdruck, entspannt. Der Druck in den Mitteln zum Speichern von Gas liegt somit in den aufeinander folgenden arteriellen und venösen Phasen immer unterhalb des vorgegebenen Drucks, insbesondere des Umgebungsdrucks, auf das das abgeschlossene System entspannt worden ist.

Während der arteriellen Phase stehen die Mittel zum Speichern von Gas nicht im direkten Kontakt mit dem Patienten, da der extrakorporale Blutkreislauf auf der venösen Seite beispielsweise mit einer Schlauchklemme abgetrennt ist. Somit spielt der Druckverlauf in den Mitteln zum Speichern von Gas während der arteriellen Phase für die Patientensicherheit eher weniger eine Rolle. Entscheidend ist aber, dass zum Ende der arteriellen Phase, d.h. zu Beginn der venösen Phase, der gewünschte Rückgabedruck in den Mitteln zum Sammeln von Blut vorliegt. Dies wird vorzugsweise dadurch erreicht, dass die arterielle Phase in zwei Zeitintervalle unterteilt wird. In einem vorgegebenen ersten Zeitintervall wird eine Verbindung zwischen den Mitteln zum Sammeln von Blut und den Mitteln zum Speichern von Gas hergestellt, so dass die beim Befüllen der Mittel zum Sammeln von Blut verdrängte Luft in die Mittel zum Speichern von Gas gelangt, wodurch sich dort ein bestimmter Druck aufbaut. In einem vorgegebenen zweiten Intervall wird die Verbindung zwischen den Mitteln zum Sammeln von Blut und Speichern von Gas jedoch unterbrochen. Dadurch bleibt der Druck in den Mitteln zum Speichern von Gas konstant, während sich der Druck in den Mitteln zum Sammeln von Blut weiter erhöht, da in der arteriellen Phase Blut kontinuierlich in die Mittel zum Sammeln von Blut gefördert wird. Dadurch entstehen zwei getrennte Luftvolumina.

Bei einer bevorzugten Ausführungsform der erfindungsgemäßen Blutbehandlungsvorrichtung weist der zweite Verbindungspfad zwischen den Mitteln zum Speichern von Gas und Sammeln von Blut eine Leitung zum Fördern von Gas auf, die die Mittel zum Speichern von Gas mit den Mitteln zum Sammeln von Blut verbindet. Die Mittel zum Verdichten von Gas, beispielsweise ein Kompressor, sind in dieser Leitung angeordnet. Der erste Verbindungspfad weist eine die Mittel zum Verdichten von Gas umgehende Bypass-Leitung auf, in der ein Bypass-Ventil angeordnet ist. Wenn die Mittel zum Verdichten von Gas nicht betrieben werden, ist bei geschlossenem Bypass-Ventil das abgeschlossene Volumen der Mittel zum Sammeln von Blut von dem abgeschlossenen Volumen der Mittel zum Speichern von Gas getrennt.

Wenn das Bypass-Ventil hingegen geöffnet ist, bilden beide Volumina ein gemeinsames abgeschlossenes Volumen.

Bei einer weiteren bevorzugten Ausführungsform weist die Leitung zum Fördern von Gas einen ersten Leitungsabschnitt auf, der die Mittel zum Sammeln von Blut mit der Druckseite der Mittel zum Verdichten von Gas verbindet, und einen zweiten Leitungsabschnitt auf, der die Saugseite der Mittel zum Verdichten von Gas mit den Mitteln zum Speichern von Gas verbindet.

Für die Entspannung des abgeschlossenen Volumens auf den Umgebungsdruck bei der Initialisierung des Systems sind vorzugsweise in dem ersten Leitungsabschnitt Mittel zum Be- oder Entlüften vorgesehen.

Vorzugsweise sind die Mittel zum Sammeln von Blut als Behälter mit einem vorgegebenen Volumen ausgebildet, der einen Einlass und einen Auslass aufweist und in der Blutrückführleitung des extrakorporalen Kreislaufs angeordnet ist. Auch die Mittel zum Speichern von Gas sind vorzugsweise als ein Behälter mit einem vorgegebenen Volumen ausgebildet.

Das Blut im extrakorporalen Kreislauf wird vorzugsweise mit einer Blutpumpe gefördert, die in der Blutzuführleitung angeordnet ist.

Ein besonderer Vorteil der erfindungsgemäßen Blutbehandlungsvorrichtung liegt darin, dass ohne Niveausensoren der Füllstand der Mittel zum Sammeln von Blut möglichst genau allein auf der Grundlage von Druckmessungen ermittelt werden kann. Eine bevorzugte Ausführungsform der erfindungsgemäßen Blutbehandlungsvorrichtung weist Mittel zum Messen des Drucks in dem abgeschlossenen Volumen der Mittel zum Sammeln von Blut, dem abgeschlossenen Volumen in den Verbindungspfaden zwischen den Mitteln zum Sammeln von Blut einerseits und den Mitteln zum Verdichten von Gas andererseits und dem abgeschlossenen Volumen der Mittel zum Speichern von Gas auf, wobei das Blutvolumen aus den gemessenen Druckwerten berechnet wird.

Zur Bestimmung des Drucks in den Verbindungspfaden zwischen den Mitteln zum Sammeln von Blut einerseits und den Mitteln zum Verdichten von Gas andererseits kann der Druck entweder in einem Leitungsabschnitt des ersten Verbindungspfads und/oder in einem Leitungsabschnitt des zweiten Verbindungspfads gemessen werden, wobei sich die Leitungsabschnitte zwischen den Mitteln zum Sammeln von Blut einerseits und den Mitteln zum Verdichten von Gas bzw. den Mitteln zum Unterbrechen der Verbindung andererseits erstrecken. Wenn der erste und zweite Verbindungspfad einen gemeinsamen Leitungsabschnitt haben, ist es auch möglich, mit einem Drucksensor den Druck in dem gemeinsamen Leitungsabschnitt der beiden Verbindungspfade zu messen.

Im Folgenden wird ein Ausführungsbeispiel der Erfindung unter Bezugnahme auf die Zeichnungen näher erläutert.

Es zeigen:

Fig. 1    ein Ausführungsbeispiel der erfindungsgemäßen Blutbehandlungsvorrichtung für den Einnadel-Betrieb in stark vereinfachter schematischer Darstellung,

Fig. 2    den Verlauf des Füllstandes und des Drucks während der Initialisierung und des Betriebs der erfindungsgemäßen Blutbehandlungsvorrichtung, und

Fig. 3    den Druckverlauf während der aufeinander folgenden arteriellen und venösen Phasen nach der Initialisierung.

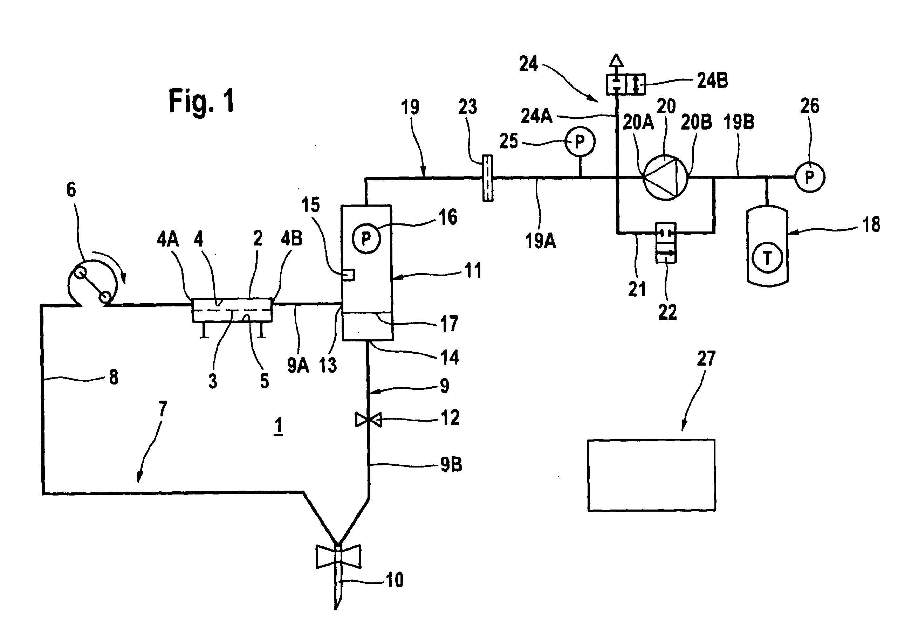

Fig. 1 zeigt die wesentlichen Komponenten einer Blutbehandlungsvorrichtung; insbesondere einer Dialysevorrichtung, für den Einnadel-Betrieb in schematischer Darstellung.

Die Dialysevorrichtung verfügt während der Blutbehandlung über einen extrakorporalen Blutkreislauf 1, der eine als Disposable ausgebildete Blutbehandlungseinheit 2, beispielsweise einen Dialysator, umfasst. Der Dialysator 2 ist durch eine semipermeable Membran 3 in eine Blutkammer 4 und eine Dialysierflüssigkeitskammer 5 unterteilt.

Im extrakorporalen Blutkreislauf wird das Blut mittels einer Blutpumpe 6 gefördert, die Teil der Dialysevorrichtung ist. Der Dialysierflüssigkeitskreislauf ist in Figur 1 nicht dargestellt.

In die Dialysevorrichtung wird ein Schlauchset 7 eingelegt, das nach der Behandlung verworfen wird. Das Disposable 7 weist eine zu dem Einlass 4A der Blutkammer 4 des Dialysators 2 führende Blutzuführleitung 8, die in die Rollenpumpe 6 der Dialysevorrichtung eingelegt ist, und eine von dem Auslass 4B der Blutkammer abgehende Blutrückführleitung 9 auf. Blutzuführ- und -rückführleitung 8, 9 sind an einer gemeinsamen Kanüle 10 (Nadel) angeschlossen.

In der Blutrückführleitung 9 des Disposable sind Mittel 11 zum Sammeln von Blut angeordnet, die als ein Behälter mit einem vorgegebenen Volumen ausgebildet sind. Nachfolgend werden die Mittel zum Sammeln von Blut als Blutsammelbehälter oder Blutspeicher bezeichnet. Stromab des Blutsammelbehälters 11 sind an der Blutrückführleitung 9 Mittel 12 zum Unterbrechen der Blutrückführleitung, beispielsweise eine venöse Schlauchklemme angeordnet.

Der Blutsammelbehälter 11 weist einen Einlass 13 auf, zu dem ein erster Abschnitt 9A der Blutrückführleitung 9 führt, und weist einen Auslass 14 auf, von dem ein zweiter Abschnitt 9B der Blutrückführleitung 9 abgeht. Zur Detektion eines bestimmten Füllstandes im Blutsammelbehälter 11 weist die Dialysevorrichtung einen Füllstandgeber 15 auf, der detektiert, wenn der Füllstand in dem Behälter einen vorgegebenen Wert erreicht. Darüber hinaus ist ein Druckgeber 16 vorgesehen, der den Druck im Blutsammelbehälter 11 misst.

Wenn der Blutsammelbehälter 11 mit Blut befüllt ist, verbleibt oberhalb des Flüssigkeitsspiegels 17 ein bestimmtes Luftvolumen in dem Blutspeicher. Der Blutspeicher steht in Strömungsverbindung mit Mitteln 18 zum Speichern von Gas, insbesondere Luft, die als ein Behälter mit einem abgeschlossenen Volumen ausgebildet sind. Nachfolgend werden die Mittel 18 zum Speichern von Gas als Luftspeicherbehälter oder Luftspeicher bezeichnet.

Damit Blutspeicher und Luftspeicher miteinander kommunizieren können, geht von der Oberseite des Blutspeichers 11 eine Leitung 19 ab, die zu dem Luftspeicher 18 führt. In der Leitung 19 sind Mittel 20 zum Verdichten von Gas angeordnet, die beispielsweise als konventioneller Kompressor ausgebildet sein können. Solange der Kompressor nicht betrieben wird, unterbricht der Kompressor die Strömungsverbindung zwischen Blutspeicher und Luftspeicher. Beim Betrieb des Kompressors wird hingegen in dem Luftspeicher befindliche Luft in den Blutspeicher überführt. Da die Luft komprimiert wird, baut sich in dem Blutspeicher ein vorgegebener Druck auf.

Die Leitung 19 weist zwei Leitungsabschnitte 19A, 19B auf, von denen der eine Leitungsabschnitt 19A den Blutspeicher 11 mit dem druckseitigen Anschluss 20A des Kompressors 20 und der andere Leitungsabschnitt 19B den saugseitigen Anschluss 20B des Kompressors 20 mit dem Luftspeicher 18 verbindet. Diese Leitungsabschnitte 19A, 19B bilden einen Verbindungspfad zum Überführen von Gas aus dem Luftspeicher in den Blutspeicher.

Um bei nicht im Betrieb befindlichem Kompressor Luft aus dem Blutspeicher in den Luftspeicher überführen zu können, ist eine Bypassleitung 21 vorgesehen, die von dem ersten Leitungsabschnitt 19A der Leitung 19 abgeht und zu dem zweiten Leitungsabschnitt 19B der Leitung 19 führt. In die Bypassleitung 21 ist ein Bypassventil 22 geschaltet. Zusammen mit den entsprechenden Leitungsabschnitten der Leitung 19 bildet die Bypassleitung 21 einen Verbindungspfad zum Fördern von Gas aus dem Blutspeicher in den Luftspeicher.

Um zu verhindern, dass Flüssigkeit aus dem Blutspeicher in den Luftspeicher gelangen kann, ist in dem ersten Leitungsabschnitt 19A der Leitung 19 ein Filter 23 angeordnet, der eine hydrophobe, d.h. für Luft durchlässige, aber für Flüssigkeit undurchlässige Membran enthält. Da der Blutspeicher nur bis zu einem maximalen Füllstand befüllt wird, kann aber ohnehin nur im Falle einer Störung Flüssigkeit in die Leitung 19 gelangen.

Zum Be- / Entlüften des abgeschlossenen Volumens, das den Blutspeicher und den Luftspeicher sowie die Leitung 19 umfasst, sind Mittel 24 zum Be- / Entlüften vorgesehen, die eine beispielsweise an den ersten Leitungsabschnitt 19A der Leitung 19 angeschlossene Be- / Entlüftungsleitung 24A mit einem Be- / Entlüftungsventil 24B aufweisen. Die Be- / Entlüftungsleitung 24A kann grundsätzlich von jeder Stelle des zu be- oder entlüftenden Volumens abgehen. Insbesondere sollte die Be- /Entlüftung im maschinenseitigen Teil erfolgen.

Neben dem Druckgeber 16 zum Messen des Drucks in dem Blutspeicher ist ein Druckgeber 25 zum Messen des Drucks in dem ersten Leitungsabschnitt 19A der Leitung 19 zwischen dem Filter 23 und dem Kompressor 20 und ein weiterer Druckgeber 26 zum Messen des Drucks im Luftspeicher 18 vorgesehen. Im Luftspeicher ist ein Temperatursensor T zum Messen der Temperatur der im Luftspeicher befindlichen Luft vorgesehen.

Die Dialysevorrichtung verfügt über eine zentrale Steuer- und Recheneinheit 27, die über nicht dargestellte elektrische Leitungen mit der Blutpumpe 6, der venösen Schlauchklemme 12, dem Bypassventil 22, dem Be- / Entlüftungsventil 24B, dem Füllstandsgeber 15, dem Kompressor 20 sowie den Druckgebern 16, 25 und 26 verbunden ist. Die zentrale Steuer- und Recheneinheit steht wiederum mit einer nicht dargestellten Eingabeeinheit in Verbindung, die eine Schnittstelle zwischen dem Benutzer und der Maschine schafft.

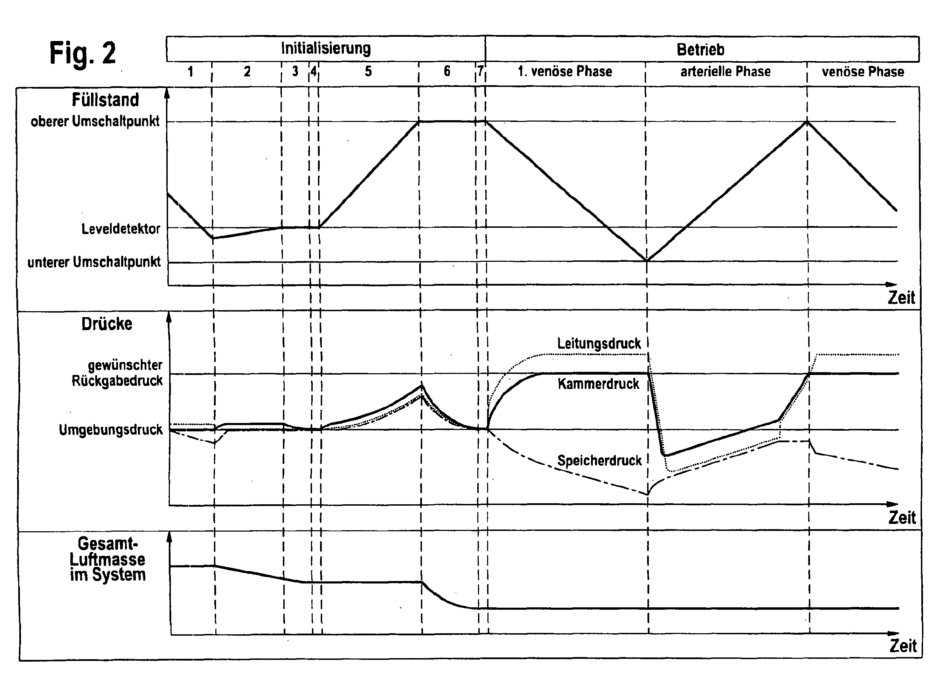

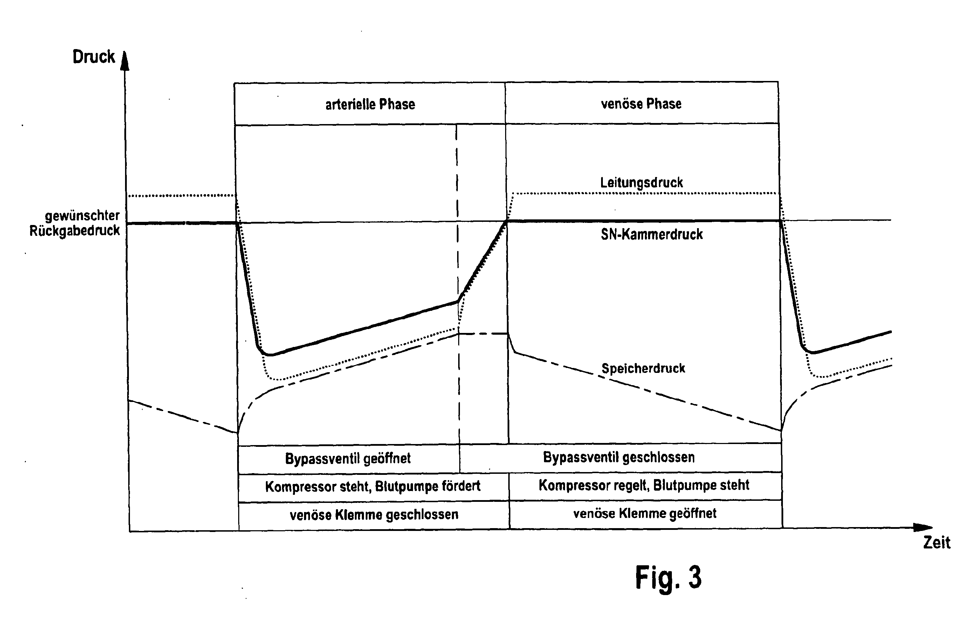

Nachfolgend wird der Betrieb der Dialysevorrichtung unter Bezugnahme auf die Figuren 2 und 3 im Einzelnen beschrieben. Die zentrale Steuer- und Recheneinheit 27 steuert die Dialysemaschine wie folgt.

Zu Beginn der eigentlichen Dialysebehandlung wird eine Initialisierung des Systems mit den folgenden Verfahrensschritten durchgeführt.

Fig. 2 zeigt den Füllstand in dem Blutspeicher als Funktion der Zeit während der einzelnen Phasen der Initialisierung. Darüber hinaus zeigt Fig. 2 den Verlauf des Drucks im Blutspeicher, der als Kammerdruck bezeichnet wird, des Drucks in dem Luftspeicher, der als Speicherdruck bezeichnet wird, und des Drucks in dem ersten Leitungsabschnitt 19A der Leitung 19, der als Leitungsdruck bezeichnet wird. Des Weiteren zeigt Figur 2 den zeitlichen Verlauf der im Blutspeicher und im Luftspeicher sowie den entsprechenden Abschnitten der Leitungen 19, 22 eingeschlossenen Gesamt-Luftmasse.

In dem ersten Initialisierungsschritt wird der Blutpegel im Blutspeicher unterhalb eines bestimmten Niveaus abgesenkt, das zwischen dem oberen Umschaltpunkt, bei dem während des Betriebs der Dialysevorrichtung von der arteriellen Phase auf die venöse Phase umgeschaltet werden soll und dem unteren Umschaltpunkt liegt, bei dem von der venösen auf die arterielle Phase umgeschaltet werden soll. Hierzu öffnet die Steuer- und Recheneinheit 27 bei stillstehender Blutpumpe 6 die venöse Schlauchklemme 12 und setzt den Kompressor 20 so lange in Betrieb, bis das gewünschte Flüssigkeitsniveau erreicht ist, das der Füllstandgeber detektiert. Fig. 2 zeigt, dass der Füllstand absinkt, während die Gesamt-Luftmasse im System konstant bleibt. Dieser Schritt kann übersprungen werden, wenn sich der Blutpegel bereits unterhalb des gewünschten Niveaus befindet.

In dem zweiten Schritt wird der Blutpegel dann auf das gewünschte Niveau eingestellt, das der Füllstandsgeber detektiert. Hierzu wird das Bypassventil 22 und das Be- / Entlüftungsventil 24B geöffnet und die Blutpumpe 6 bei geschlossener venöser Schlauchklemme so lange betrieben, bis das gewünschte Niveau erreicht ist. Fig. 2 zeigt, dass der Füllstand auf das gewünschte Niveau ansteigt, während die Luftmasse im System abnimmt.

Wenn das gewünschte Niveau erreicht ist, wird so lange gewartet, bis sich der Kammer-und Speicherdruck sowie der Leitungsdruck auf Umgebungsdruck eingestellt haben. Dabei bleibt der Füllstand konstant, während die Luftmasse weiter leicht abnimmt (Schritt 3). Erst dann wird das Be- / Entlüftungsventil 24B wieder geschlossen (Schritt 4).

Daraufhin wird der Blutspeicher weiter mit Blut befüllt. Bei geöffnetem Bypassventil wird die Blutpumpe 6 solange betrieben, bis der Füllstand im Blutspeicher das Niveau des oberen Umschaltpunktes erreicht hat (Schritt 5). Dabei bleibt die Luftmasse im System konstant. Da der Kammer- und Speicherdruck sowie der Leistungsdruck mit den Druckgebern gemessen werden, kann der Füllstand im Blutspeicher fortlaufend berechnet werden. Die Steuer- und Recheneinheit berechnet den Füllstand im Blutspeicher und hält die Blutpumpe dann an, wenn der Füllstand das Niveau des oberen Umschaltpunktes erreicht hat. Dies wird später noch im Einzelnen beschrieben.

Nachdem die Steuer- und Recheneinheit die Blutpumpe 6 gestoppt hat, wird das Be- / Entlüftungsventil 24B wieder geöffnet, so dass sich der im System aufgebaute Druck auf den Umgebungsdruck entspannt (Schritt 6). Fig. 2 zeigt, dass der Füllstand konstant bleibt, während der Kammer- und Speicherdruck sowie der Leitungsdruck auf den Umgebungsdruck absinken. Da das Volumen, das von dem System eingeschlossen wird, d.h. sowohl das Volumen des Blutspeichers und des Luftspeichers sowie der Leitungen als auch die Drücke im System bekannt sind, kann die im System enthaltene Luftmasse berechnet werden. Dies wird später noch im Einzelnen beschrieben.

Als letzter Schritt der Initialisierung wird das Be- / Entlüftungsventil 24B geschlossen, wobei sich weder der Füllstand noch die Drücke sowie die Luftmasse im System verändern (Schritt 7). Das Be- / Entlüftungsventil bleibt während der gesamten Blutbehandlung geschlossen, wenn nicht eine erneute Initialisierung beispielsweise nach der Detektion einer Luftleckage erforderlich ist. Damit ist die Initialisierung abgeschlossen, und die Blutbehandlung startet mit der ersten venösen Phase.

In der ersten venösen Phase wird der Kompressor 20 bei geschlossenem Bypassventil 22 betrieben, wobei die venöse Schlauchklemme 12 geöffnet ist und die Blutpumpe 6 still steht. Während des Betriebs des Kompressors wird Luft aus dem Luftspeicher 18 komprimiert und dem Blutspeicher 11 zugeführt. Dadurch nehmen Kammer- und Leitungsdruck zu, während der Speicherdruck abnimmt. Gleichzeitig nimmt der Füllstand im Blutspeicher kontinuierlich ab, bis das Niveau des unteren Umschaltpunkts erreicht ist. Dabei ist entscheidend, dass der Speicherdruck unterhalb des Kammerdrucks und folglich auch unterhalb des Leitungsdrucks liegt, um im Falle eines Fehlers einen Gaseintrag von dem Luftspeicher in den Blutspeicher zu verhindern. Zusätzlich wird angestrebt, dass der Speicherdruck sogar unterhalb des Umgebungsdrucks liegt.

Daraufhin beginnt die arterielle Phase, in der der Blutspeicher wieder mit dem Patienten entnommenen Blut befüllt wird, woraufhin sich wieder die venöse Phase anschließt, in der das Blut aus dem Blutspeicher wieder dem Patienten zugeführt wird.

Der Luftspeicher ist so groß dimensioniert, dass auch am Ende der venösen Phase genügend Luft im System vorhanden ist, um den gewünschten Rückgabedruck in dem Blutspeicher aufrechterhalten zu können. Um mit der gleichen Initialisierung sämtliche Betriebspunkte mit einem Rückgabedruck von 0 bis 500 mmHg relativ bei einem Schlagvolumen bis 60 ml einstellen zu können, wird in der Praxis ein Luftspeicher mit einem Speichervolumen von ca. 300 ml benötigt.

Der zeitliche Verlauf des Kammer- und Speicherdrucks sowie des Leitungsdrucks während der eigentlichen arteriellen und venösen Phasen nach der Initialisierung des Systems ist in Fig. 3 dargestellt, die einen Ausschnitt von Fig. 2 zeigt.

Während der gesamten arteriellen Phase wird die Blutpumpe 6 betrieben, wobei der Kompressor 20 stillsteht. Die venöse Schlauchklemme 12 bleibt während der gesamten arteriellen Phase geschlossen.

Zu Beginn der arteriellen Phase öffnet die Steuer- und Recheneinheit 27 das Bypassventil 22, so dass die aus dem Blutspeicher 11 verdrängte Luft über die Bypassleitung 21 in den Luftspeicher 18 gelangt. Folglich steigt der Speicherdruck an, während der Kammer- und Leitungsdruck zunächst absinkt, um dann ebenfalls gleichsam mit dem Speicherdruck anzusteigen. Die im Blutspeicher und in dem zugehörigen Leitungsvolumen enthaltene Luftmasse nimmt damit kontinuierlich ab.

Sobald die im Blutspeicher und dem Leitungsvolumen enthaltene Luftmasse einen vorgegebenen Betrag erreicht hat, der sich aus dem gewünschten Schlagvolumen und dem gewünschten Rückgabedruck ergibt, schließt die Steuer- und Recheneinheit das Bypassventil. Folglich entstehen zwei getrennte Luftvolumina, d.h. das Luftvolumen des Blutspeichers mit den zugehörigen Leitungsabschnitten und das Volumen des Luftspeichers mit den zugehörigen Leitungsabschnitten. Daraufhin wird die Blutpumpe bei geschlossenem Bypassventil betrieben, so dass der Speicherdruck konstant bleibt, während die Luft in dem Blutspeicher und dem zugehörigen Leitungsvolumen so lange verdichtet wird, bis bei Erreichen des gewünschten Schlagvolumens auch der gewünschte Rückgabedruck erreicht ist. Figur 3 zeigt, dass zum Ende der arteriellen Phase der Kammer- und Leitungsdruck auf den gewünschten Rückgabedruck angestiegen sind, wobei der Speicherdruck während der gesamten arteriellen Phase immer unterhalb des Kammer- und Leitungsdrucks, insbesondere unterhalb des Umgebungdrucks liegt. Dadurch ist ausgeschlossen, dass auch bei einem Störfall des Systems, beispielsweise bei einer Leckage des Kompressors, Luft aus dem Luftspeicher in den Blutspeicher gelangt.

Anstelle der Unterteilung der arteriellen Phase in ein erstes und zweites Zeitintervall sind auch andere Ausführungsformen möglich, mit denen sich auch die erfindungsgemäßen Vorteile erzielen lassen. Eine alternative Ausführungsform sieht vor, anstelle des Bypassventils 22 ein druckgesteuertes Ventil einzusetzen, das bei Erreichen eines dem Rückgabedruck entsprechenden Grenzdrucks öffnet, so dass in einem gewissen Zeitbereich ein konstanter Druck im Blutspeicher herrscht.

Anschließend schaltet die Steuer- und Recheneinheit auf die venöse Phase um, wobei das Bypassventil geschlossen bleibt, die Blutpumpe angehalten und der Kompressor in Betrieb gesetzt sowie die venöse Schlauchklemme geöffnet wird. Der Kompressor wird während der gesamten venösen Phase betrieben, wobei die Blutpumpe stillsteht. Während der venösen Phase bleibt die venöse Schlauchklemme geöffnet und das Bypassventil geschlossen.

Der Kompressor fördert Luft aus dem Luftspeicher in den Blutspeicher, um einen Überdruck aufzubauen, so dass Blut aus dem Blutspeicher gefördert wird. Der Kompressor wird dabei derart betrieben, dass sich im Blutspeicher der gewünschte Rückgabedruck einstellt. Da dem Blutspeicher fortwährend Luft aus dem Luftspeicher zugeführt wird, nimmt der Speicherdruck kontinuierlich ab. Entscheidend ist wieder, dass der Speicherdruck immer unterhalb des Kammer- und Leitungsdrucks, vorzugsweise auch unterhalb des Umgebungdrucks liegt, so dass eine Luftinfusion aus dem Luftspeicher in den Patienten bei einem Störfall ausgeschlossen ist. Die venöse Phase ist dann beendet, wenn der Blutpegel im Blutspeicher wieder auf das Niveau des unteren Umschaltpunkts abgesunken ist. Dann schließt sich die nächste arterielle Phase an.

Nachfolgend wird die Berechnung des im Blutspeicher befindlichen Blutvolumens $V_{Blut}$ beschrieben, die von der Steuer- und Recheneinheit während des Betriebs der Dialysevorrichtung kontinuierlich oder in vorgegebenen Intervallen vorgenommen wird. Wenn das Blutvolumen bekannt ist, kann dies zum Vergleich mit den Umschaltpunkten von der arteriellen auf die venöse Phase oder umgekehrt verwendet werden.

Zur Berechnung des in dem Blutspeicher befindlichen Blutvolumens $V_{Blut}$ ist es zunächst notwendig, die Luftmasse im Blutspeicher zu bestimmen. Nach erfolgtem Druckausgleich gilt:

$$pV = \frac{m}{M_m} RT$$

mit:

p = Druck (absolut), V = Volumen, m = Masse, $M_m$ = molare Masse,
R = allg. Gaskonstante und T = Temperatur

Da $M_m$ und $R$ konstant sind und für die Luftmasse keine absoluten Werte benötigt werden, brauchen diese nicht explizit berücksichtigt zu werden. Bestimmt werden muss demnach nur

$$\frac{pV}{T} \equiv m.$$

Zur Bestimmung der gesamten Luftmasse muss die Summe aller Teilluftmassen, d.h. der Luftmasse im Blutspeicher, den zugehörigen Leitungen und im Luftspeicher gebildet werden. Hierzu müssen alle mit Luft gefüllten Volumina mit dem jeweils herrschenden Druck multipliziert und durch die Temperatur dividiert werden.

$$\left(\frac{pV}{T}\right)_{ges} = \frac{p_{Blutspeicher} \cdot V_{Blutspeicher / Luft}}{T_{Blutspeicher}} + \frac{p_{Leitung} \cdot V_{Leitung}}{T_{Leitung}} + \frac{p_{Luftspeicher} \cdot V_{Luftspeicher}}{T_{Luftspeicher}}$$

$$T_{Blutspeicher} \approx 273{,}15 \text{ K} + 36\text{K}$$

$$T_{Leitung} \approx \frac{T_{Blutspeicher} + T_{Luftspeicher}}{2}$$

Das Luftvolumen des Blutspeichers $V_{Blutspeicher/Luft}$ ändert sich während des Betriebs. Ist der Blutpegel auf der Höhe H des von dem Füllstandgeber 15 detektierten Niveaus, so entspricht

$$V_{Blutspeicher/Luft} = V_{Blutspeicher/Luft\,UT} - \Delta\,V_{Blutspeicher/\,UT-H,}$$

wobei $V_{Blutspeicher/Luft\,UT}$ das Luftvolumen im Blutspeicher am unteren Umschaltpunkt UT und $\Delta\,V_{Blutspeicher/UT-H}$ die Volumendifferenz zwischen dem unteren Umschaltpunkt UT und der Höhe des von dem Füllstandgeber 15 detektierten Füllstands im Blutspeicher ist.

Am Ende der Initialisierung am oberen Umschaltpunkt OT befindet sich zusätzlich Blut mit dem gesamten Schlagvolumen $V_{Schlag}$ in dem Blutspeicher, so dass

$$V_{Blutspeicher/Luft} = V_{Blutspeicher/Luft\,UT} - V_{Schlag} \text{ ist.}$$

Die restlichen Volumina bleiben konstant. Dabei sind die Temperaturen in guter Näherung als konstant zu setzen, wenn keine Temperaturkompensation erfolgt. Vorzugsweise ist aber ein Temperatursensor zur Temperaturmessung zumindest im Luftspeicher vorgesehen, so dass eine Temperaturkompensation vorgenommen werden kann. Es können aber auch Temperatursensoren für die anderen Druckwerte vorgesehen sein.

Das geförderte Blutvolumen $V_{Blut}$ im Blutspeicher wird während des gesamten Zyklus bei jeder Programmschleife berechnet. Dabei wird anhand der gemessenen Drücke das im Blutspeicher eingeschlossene Luftvolumen $V_{Blutspeicher/Luft}$ berechnet und die Differenz von dem Luftvolumen im Blutspeicher bei dem unteren Umschaltpunkt UT und dem Luftvolumen des Blutspeichers gebildet.

$$V_{Blutspeicher/Luft} = \frac{\left(\frac{pV}{T}\right)_{ges} - \frac{P_{Leitung}V_{Leitung}}{T_{Leitung}} - \frac{P_{Luftspeicher}V_{Luftspeicher}}{T_{Luftspeicher}}}{\frac{P_{Blutspeicher}}{T_{Blutspeicher}}}$$

$$V_{Blut} = V_{Blutspeicher/Luft\,UT} - V_{Blutspeicher/Luft}$$

Die Gesamtluftmasse $\left(\frac{pV}{T}\right)_{ges}$ bleibt nach der Initialisierung des Systems unverändert, weil das Entlüftungsventil geschlossen bleibt. Da die Berechnung des Blutvolumens $V_{Blut}$ bei laufender Blutpumpe bzw. laufendem Kompressor erfolgt, wird eine Glättung der Drucksignale und des berechneten Blutvolumens $V_{Blut}$ durchgeführt.

Die Umschaltung von arterieller auf venöse Phase (OT) erfolgt, wenn die Differenz von dem berechneten Blutvolumen $V_{Blut}$ und dem eingestellten Schlagvolumen $V_{Schlag}$ gleich Null ist, und die Umschaltung von venöser auf arterielle Phase erfolgt, wenn das berechnete Blutvolumen gleich Null ist.

Im Folgenden wird beschrieben, wie die Steuer- und Recheneinheit den Zeitpunkt berechnet, zu dem innerhalb der arteriellen Phase zwischen dem ersten und zweiten Zeitintervall der arteriellen Phase umgeschaltet wird.

Wie bereits oben beschrieben, ist der Umschaltpunkt von arterieller zu venöser Phase und zurück durch einen einfachen Vergleich des Blutvolumens mit dem Schlagvolumen bzw. Null möglich. Die arterielle Phase teilt sich, wie bereits erwähnt, in ein erstes und ein zweites Zeitintervall auf. In der ersten arteriellen Phase fördert die Blutpumpe Blut über den Dialysator bei geöffnetem Bypassventil in den Blutspeicher. Dadurch wird der Unterdruck, der sich in der venösen Phase zuvor im Luftspeicher aufgebaut hat, zur Unterstützung der Pumpe genutzt. In der zweiten arteriellen Phase wird der Luftspeicher durch Schließen des Bypassventils von dem übrigen System abgekoppelt; und der Druck in dem Blutspeicher steigt durch das geförderte Blutvolumen stark an. Am Ende der zweiten arteriellen Phase soll im Blutspeicher der gewünschte Solldruck anstehen. Der Umschaltpunkt zwischen erster und zweiter arterieller Phase muss daher so gewählt werden, dass das noch ausstehende Blutvolumen bis zum oberen Umschaltpunkt OT in dem Blutspeicher und der Leitung den Solldruck aufbaut.

Berechnet werden muss demnach die Luftmasse, die bei Kompression auf das bei dem oberen Umschaltpunkt OT in dem Blutspeicher und der Leitung verfügbare Luftvolumen den Solldruck $p_{soll}$ aufbaut. Die Luftmasse in Blutspeicher und Leitung ist

$$\frac{p_{Blutspeicher}V_{Blutspeicher}}{T_{Blutspeicher}} + \frac{p_{Leitung}V_{Leitung}}{T_{Leitung}} = \left(\frac{pV}{T}\right)_{ges} - \frac{p_{Luftspeicher}V_{Luftspeicher}}{T_{Luftspeicher}}.$$

Beim Umschaltpunkt von dem ersten auf das zweite Zeitintervall der arteriellen Phase muss die in Blutspeicher und Leitung befindliche Luftmasse gleich der Luftmasse sein, die sich im oberen Umschaltpunkt OT in dem Blutspeicher und der Leitung befindet.

$$\left(\frac{pV}{T}\right)_{ges} - \frac{p_{Luftspeicher}V_{Luftspeicher}}{T_{Luftspeicher}} = \frac{p_{Soll} \cdot V_{Blutspeicher / LuftOT}}{T_{Blutspeicher}} + \frac{p_{Soll} \cdot V_{Leitung}}{T_{Leitung}}$$

Dabei ist $V_{Blutspeicher/LuftOT}$ das Luftvolumen im Blutspeicher am oberen Umschaltpunkt OT.

Die Steuer- und Recheneinheit überprüft während der arteriellen Phase, ob die obige Gleichung erfüllt ist. Sobald die Gleichung erfüllt ist, beginnt das zweite Zeitintervall der arteriellen Phase, wobei das Bypassventil geschlossen wird. Die Blutpumpe wird von der Steuer- und Recheneinheit in dem zweiten Zeitintervall mit der gleichen Förderrate betrieben, bis das gewünschte Schlagvolumen und somit der obere Umschaltpunkt OT erreicht ist.

Eine Leckage in dem abgeschlossenen Volumen kann eine Veränderung der im System eingeschlossenen Gasmenge zur Folge haben. Wenn das Leck im Überdruckbereich des Systems liegt, d.h. in dem Bereich des Blutspeichers oder der angrenzenden Leitungsabschnitte, so führt das Leck zu Verringerung der eingeschlossenen Luftmenge, wodurch der Füllstand des Bluts in dem Blutspeicher ansteigt. Dabei besteht die Gefahr, dass der Blutspeicher vollläuft und der Blutpegel bis zu der Hydrophobmembran ansteigt, so dass die arterielle Phase nicht mehr ordnungsgemäß beendet werden kann. Umgekehrt wird bei einer Leckage im Unterdruckbereich, d.h. im Gasspeicher oder durch Lufteintrag aus dem Blutsystem die eingeschlossene Luftmenge vergrößert, so dass der Füllstand des Bluts im Blutspeicher sinkt. Das kann dazu führen, dass der Blutpegel in der venösen Phase zu schnell abfällt, was zu unerwünschter Schaumbildung, im Extremfall sogar zum Luftalarm führen kann. Dies kann aber dadurch überwacht werden, dass die im System eingeschlossene Luftmenge beobachtet wird wird. Eine derartige Überwachung der Luftmenge kann bei der erfindungsgemäßen Vorrichtung vorgesehen sein.

Die Überwachung der Luftmenge zur Erkennung einer Leckage erfolgt dadurch, dass der Zeitpunkt erfasst wird, zu dem der Füllstandgeber 15 im Blutspeicher 11 den Füllstand detektiert, d.h. das Blut den vorgegebenen Pegel erreicht. Damit ist für diesen Zeitpunkt der tatsächliche Füllstand des Bluts bekannt. Dieser Wert wird mit dem zu diesem Zeitpunkt mit dem aus den Druckwerten berechneten Füllstand verglichen. Wenn die Differenz von dem gemessenen und berechneten Füllstand größer als ein vorgegebener Grenzwert ist, hat sich die in dem System eingeschlossene Luftmenge signifikant verändert, was auf eine Leckage im System zurückgeführt werden kann. In diesem Fall wird das System neu initialisiert. Tritt der Fehlerfall zu häufig auf, bricht die zentrale Steuer- und Recheneinheit 27 die Behandlung ab.

Eine alternative Auswertung sieht vor, nicht auf den gemessenen und berechneten Füllstand abzustellen, sondern den Zeitpunkt, zu dem der Füllstandgeber 15 den vorgegebenen Füllstand detektiert, mit dem berechneten Zeitpunkt zu vergleichen, zu dem sich der vorgegebene Füllstand einstellen sollte. Liegt eine signifikante Zeitdifferenz vor, wird auf eine Leckage im System geschlossen.

Eine weitere Ausführungsform sieht zusätzlich vor, eine Leckage im System in dem dem Blutspeicher 11 abgewandten Teil hinter dem Filter 23 nach dem Ansteigen des Füllstands über das vom Füllstandgeber detektierte Niveau in der arteriellen Phase dadurch zu erkennen, dass mit dem Drucksensors 16 in dem Blutspeicher 11 ein zu stark ansteigender Druck erkannt wird, wobei der Druck und/oder der Druckanstieg pro Zeiteinheit einen vorgegebenen Grenzwert überschreitet. In diesem Fall führt das den Filter erreichende Blut zu einem Druckanstieg, was die Compliance in diesem Teil des Systems drastisch verringert. Dies trifft insbesondere für den Fall zu, in dem ein Drucksensor verwendet wird, der den Druck in direktem Kontakt, d.h. ohne kompressiblen Zwischenraum, misst.

**Patentansprüche**

1.  Vorrichtung zur Blutbehandlung im Einnadel-Betrieb mit
    Mitteln (6) zum Fördern von Blut in einem extrakorporalen Blutkreislauf zu Mitteln (11) zum Sammeln von Blut, wobei der extrakorporale Blutkreislauf eine zu einem Einlass (4A) einer Blutbehandlungseinheit (2) führende Blutzuführleitung (8) und eine von einem Auslass (4B) der Blutbehandlungseinheit abgehende Blutrückführleitung (9) aufweist, und die Mittel zum Sammeln von Blut ein abgeschlossenes Volumen umfassen,
    Mitteln (18) zum Speichern von Gas, die ein abgeschlossenes Volumen umfassen,
    einem ersten Verbindungspfad (21) zum Verbinden des abgeschlossenen Volumens der Mittel (11) zum Sammeln von Blut mit dem abgeschlossenen Volumen der Mittel (18) zum Speichern von Gas derart, dass in einer arteriellen Phase beim Befüllen der Mittel zum Sammeln von Blut aus den Mitteln zum Sammeln von Blut verdrängtes Gas zu den Mitteln zum Speichern von Gas überführt wird, wobei der erste Verbindungspfad (21) Mittel (22) zum Unterbrechen der Verbindung enthält,
    einem Mittel zum Verdichten (20) von Gas enthaltenden zweiten Verbindungspfad (19) zum Verbinden des abgeschlossenen Volumens der Mittel (18) zum Speichern von Gas mit dem abgeschlossenen Volumen der Mittel (11) zum Sammeln von Blut derart, dass in einer venösen Phase zum Entleeren der Mittel zum Sammeln von Blut in den Mitteln zum Speichern von Gas gespeichertes Gas mit den Mitteln zum Verdichten von Gas zu den Mitteln zum Sammeln von Blut überführt wird,
    so dass dem extrakorporalen Blutkreislauf in der arteriellen Phase Blut zugeführt und in der venösen Phase von dem extrakorporalen Blutkreislauf Blut abgeführt werden kann.

2.  Vorrichtung zur Blutbehandlung im Einnadel-Betrieb nach Anspruch 1, **dadurch gekennzeichnet, dass** die Vorrichtung eine Blutbehandlungseinheit (2) mit einem Einlass (4A) und einem Auslass (4B) und einen extrakorporalen Blutkreislauf (1) mit einer zu dem Einlass der Blutbehandlungseinheit führenden Blutzuführleitung (8) und einer von dem Auslass der Blutbehandlungseinheit abgehenden Blutrückführleitung (9) aufweist, wobei die Mittel zum Sammeln von Blut über den ersten Verbindungspfad mit den Mitteln zum Speichern von Gas in Verbindung stehen, und die Mittel zum Speichern von Gas über den zweiten Verbindungspfad mit den Mitteln zum Sammeln von Blut in Verbindung stehen.

3.  Blutbehandlungsvorrichtung nach Anspruch 1 oder 2, **dadurch gekennzeichnet, dass** der erste Verbindungspfad eine die Mittel (20) zum Verdichten von Gas umgehende Bypass-Leitung (21) aufweist, in der ein Bypass-Ventil (22) angeordnet ist, und der zweite Verbindungspfad eine Leitung (19) zum Fördern von Gas aufweist, die die Mittel (11) zum Sammeln von Blut mit den Mitteln (18) zum Speichern von Gas verbindet, wobei die Mittel (20) zum Verdichten von Gas in der Leitung zum Fördern von Gas angeordnet sind.

4.  Blutbehandlungsvorrichtung nach Anspruch 3, **dadurch gekennzeichnet, dass** die Leitung zum Fördern von Gas (19) einen ersten Leitungsabschnitt (19A) aufweist, der die Mittel (11) zum Sammeln von Blut mit der Druckseite der Mittel (20) zum Verdichten von Gas verbindet, und einen zweiten Leitungsabschnitt (19B) aufweist, der die Saugseite der Mittel zum Verdichten von Gas mit den Mitteln zum Speichern (18) von Gas verbindet, wobei Mittel (24B) zum Be- / Entlüften des ersten Leitungsabschnittes vorgesehen sind.

5.  Blutbehandlungsvorrichtung nach einem der Ansprüche 1 bis 4, **dadurch gekennzeichnet, dass** die Mittel (11) zum Sammeln von Blut als Behälter mit einem vorgegebenen Volumen ausgebildet sind, der in der Blutrückführleitung (9) des extrakorporalen Kreislaufs (1) angeordnet ist.

6.  Blutbehandlungsvorrichtung nach einem der Ansprüche 1 bis 5, **dadurch gekennzeichnet, dass** die Mittel (18) zum Speichern von Gas als ein Behälter mit einem vorgegebenen Volumen ausgebildet sind.

7.  Blutbehandlungsvorrichtung nach einem der Ansprüche 1 bis 6, **dadurch gekennzeichnet, dass** die Mittel (6) zum Fördern von Blut im extrakorporalen Kreislauf (1) als Blutpumpe ausgebildet sind, die in der Blutzuführleitung (8) angeordnet ist.

8.  Blutbehandlungsvorrichtung nach einem der Ansprüche 1 bis 7, **dadurch gekennzeichnet, dass** Mittel (27) zur Ermittlung des in den Mitteln (18) zum Sammeln von Blut eingeschlossenen Blutvolumens $V_{Blut}$ vorgesehen sind.

9.  Blutbehandlungsvorrichtung nach Anspruch 8, **dadurch gekennzeichnet, dass** die Mittel (27) zur Ermittlung des in den Mitteln (11) zum Sammeln von Blut eingeschlossenen Blutvolumens $V_{Blut}$ aufweisen:

Mittel (16) zum Messen des Drucks in den Mitteln (11) zum Sammeln von Blut,

Mittel (25) zum Messen des Drucks in den Verbindungspfaden (19, 21) zwischen den Mitteln (11) zum Sammeln von Blut einerseits und den Mitteln (20) zum Verdichten von Gas andererseits, und

Mittel (26) zum Messen des Drucks in den Mitteln (18) zum Speichern von Gas,

wobei die Mittel (27) zur Ermittlung des Blutvolumens $V_{Blut}$ derart ausgebildet sind, dass das Blutvolumen $V_{Blut}$ auf der Grundlage des gemessenen Drucks in dem abgeschlossenen Volumen der Mittel zum Sammeln von Blut, dem abgeschlossenen Volumen in den Verbindungspfaden (19, 21) zwischen den Mitteln (11) zum Sammeln von Blut einerseits und den Mitteln (20) zum Verdichten von Gas andererseits und dem abgeschlossenen Volumen der Mittel zum Speichern von Gas berechnet wird.

10. Blutbehandlungsvorrichtung nach einem der Ansprüche 1 bis 9, **dadurch gekennzeichnet, dass** Mittel (27) zum Umschalten zwischen der arteriellen Phase und der venösen Phase vorgesehen sind, die derart ausgebildet sind, dass in der arteriellen Phase die Mittel (6) zum Fördern von Blut im extrakorporalen Kreislauf (1) betrieben werden, wobei die Mittel (11) zum Sammeln von Blut über den ersten Verbindungspfad (21) mit den Mitteln (18) zum Speichern von Gas in Verbindung stehen, so dass beim Befüllen der Mittel zum Sammeln von Blut aus den Mitteln zum Sammeln von Blut verdrängtes Gas zu den Mitteln zum Speichern von Gas überführt wird, dass in der venösen Phase die Mittel (20) zum Verdichten von Gas betrieben werden, wobei die Mittel (18) zum Speichern von Gas mit den Mitteln (11) zum Sammeln von Blut über den zweiten Verbindungspfad (19) in Verbindung stehen, so dass zum Entleeren der Mittel zum Sammeln von Blut in den Mitteln zum Speichern von Gas gespeichertes Gas mit den Mitteln zum Verdichten von Gas zu den Mitteln zum Sammeln von Blut überführt wird.

11. Blutbehandlungsvorrichtung nach Anspruch 10, **dadurch gekennzeichnet, dass** die Mittel (27) zum Umschalten zwischen arterieller und venöser Phase derart ausgebildet sind, dass in der arteriellen Phase in einem ersten Zeitintervall die Mittel (22) zum Unterbrechen des ersten Verbindungspfades (21) derart betätigt werden, dass der erste Verbindungspfad nicht unterbrochen ist, und in einem zweiten Zeitintervall die Mittel (22) zum Unterbrechen des ersten Verbindungspfades (21) derart betätigt werden, dass der erste Verbindungspfad unterbrochen ist.

## Claims

1. A device for blood treatment in the single-needle operation with
means (6) for conveying blood in an extracorporeal blood circuit to means (11) for collecting blood, whereby the extracorporeal blood circuit has a blood supply line (8) leading to the inlet (4A) of a blood treatment unit (2) and a blood return line (9) departing from an outlet (4B) of the blood treatment unit, and the means for collecting blood comprise a closed-off volume,
means (18) for storing gas, which comprise a closed-off volume,
a first connection path (21) for connecting the closed-off volume of the means (11) for collecting blood to the closed-off volume of the means (18) for storing gas in such a way that, in an arterial phase, gas expelled from the means for collecting blood during the filling of the means for collecting blood is transferred to the means for storing gas, whereby the first connection path (21) contains means (22) for interrupting the connection,
a second connection path (19), containing means for compressing (20) gas, for connecting the closed-off volume of the means (18) for storing gas to the closed-off volume of the means (11) for collecting blood in such a way that in a venous phase, for the purpose of emptying the means for collecting blood, gas stored in the means for storing gas is transferred by the means for compressing gas to the means for collecting blood,
so that blood can be fed to the extracorporeal blood circuit in the arterial phase and removed from the extracorporeal blood circuit in the venous phase.

2. The device for blood treatment in the single-needle operation according to claim 1, **characterised in that** the device comprises a blood treatment unit (2) with an inlet (4A) and an outlet (4B) and an extracorporeal blood circuit (1) with a blood supply line (8) leading to the inlet of the blood treatment unit and a blood return line (9) departing from the outlet of the blood treatment unit, whereby the means for collecting blood via the first connection path are connected to means for storing gas, and the means for storing gas are connected via the second connection path to the means for collecting blood.

3. The blood treatment device according to claim 1 or 2, **characterised in that** the first connection path comprises a bypass line (21) bypassing the means (20) for compressing gas, in which bypass line a bypass valve (22) is disposed, and the second connection path comprises a line (19) for conveying gas, which line connects the means (11) for collecting blood to the means (18) for storing gas, the means (20) for compressing gas being disposed in the line for conveying gas.

**4.** The blood treatment device according to claim 3, **characterised in that** the line for conveying gas (19) comprises a first line segment (19A), which connects the means (11) for collecting blood to the pressure-side of the means (20) for compressing gas, and a second line segment (19B), which connects the suction-side of the means for compressing gas to the means for storing (18) gas, means (24B) being provided for the ventilation/deaeration of the first line segment.

**5.** The blood treatment device according to any one of claims 1 to 4, **characterised in that** the means (11) for collecting blood are designed as a container with a predetermined volume, which is disposed in the blood return line (9) of the extracorporeal circuit (1).

**6.** The blood treatment device according to any one of claims 1 to 5, **characterised in that** the means (18) for storing gas are designed as a container with a predetermined volume.

**7.** The blood treatment device according to any one of claims 1 to 6, **characterised in that** the means (6) for conveying blood in the extracorporeal circuit (1) are designed as a blood pump, which is disposed in the blood supply line (8).

**8.** The blood treatment device according to any one of claims 1 to 7, **characterised in that** means (27) are provided for determining blood volume $V_{blood}$ enclosed in the means (18) for collecting blood.

**9.** The blood treatment device according to claim 8, **characterised in that** the means (27) for determining blood volume $V_{blood}$ enclosed in the means (11) for collecting blood comprise:

means (16) for measuring the pressure in the means (11) for collecting blood,
means (25) for measuring the pressure in the connection paths (19, 21) between the means (11) for collecting blood on the one hand and the means (20) for compressing gas on the other hand, and
means (26) for measuring the pressure in the means (18) for storing gas,
whereby the means (27) for determining blood volume $V_{blood}$ are designed in such a way that blood volume $V_{blood}$ is calculated on the basis of the measured pressure in the closed-off volume of the means for collecting blood, the closed-off volume of the connection paths (19, 21) between the means (11) for collecting blood on the one hand and the means (20) for compressing gas on the other hand and the closed-off volume of the means for storing gas.

**10.** The blood treatment device according to any one of claims 1 to 9, **characterised in that** means (27) are provided for changing-over between the arterial phase and the venous phase, which means are designed in such a way that the means (6) for conveying blood in the extracorporeal circuit (1) are operated in the arterial phase, whereby the means (11) for collecting blood are connected via the first connection path (21) to the means (18) for storing gas, so that gas expelled from the means for collecting blood during the filling of the means for collecting blood is transferred to the means for storing gas, that the means (20) for compressing gas are operated in the venous phase, whereby the means (18) for storing gas are connected via the second connection path (19) to the means (11) for collecting blood, so that, for the purpose of emptying the means for collecting blood, gas stored in the means for storing gas is transferred by the means for compressing gas to the means for collecting blood.

**11.** The blood treatment device according to claim 10, **characterised in that** the means (27) for changing-over between the arterial phase and venous phase are designed in such a way that, in the arterial phase, the means (22) for interrupting the first connection path (21) are actuated in a first time interval in such a way that the first connection path is not interrupted, and the means (22) for interrupting the first connection path (21) are actuated in a second time interval in such a way that the first connection path is interrupted.

**Revendications**

**1.** Dispositif de traitement du sang en mode à une aiguille, comportant
des moyens (6) pour véhiculer le sang, dans un circuit sanguin extracorporel, vers des moyens (11) pour collecter le sang,
le circuit sanguin extracorporel présentant un conduit d'alimentation en sang (8), menant à une entrée (4A) d'une unité de traitement du sang (2), et un conduit de retour du sang (9) partant d'une sortie (4B) de l'unité de traitement du sang, et les moyens pour collec-ter le sang comprenant un volume clos,
des moyens (18) pour stocker un gaz, qui comprennent un volume clos,
un premier parcours de liaison (21) pour relier le volume clos des moyens (11) pour collecter le sang au volume clos des moyens (18) pour stocker un gaz, de telle sorte que, dans une phase artérielle, lors du remplissage des moyens pour collecter le sang, le gaz refoulé des moyens pour collecter le sang est transféré vers les moyens pour stocker un gaz, le premier parcours de liaison (21) contenant des moyens (22) pour interrompre la liaison,
un deuxième parcours de liaison (19) contenant des moyens (20) pour comprimer un gaz, pour relier le volume clos des moyens (18) pour stocker un gaz au volume clos des moyens (11) pour collecter le sang, de telle sorte que, dans une phase veineuse, afin de vider les moyens pour collecter le sang, le gaz stocké dans les moyens pour stocker un gaz est transféré par les moyens pour comprimer un gaz vers les moyens pour collecter le sang,
de sorte que du sang peut être apporté au cir-cuit sanguin extracorporel dans la phase artérielle et que du sang peut être évacué du circuit sanguin extracorporel dans la phase veineuse.

2. Dispositif de traitement du sang en mode à une aiguille selon la revendication 1, caracté-risé en ce que le dispositif présente une unité de traitement du sang (2) pourvue d'une entrée (4A) et d'une sortie (4B), et un circuit sanguin extracorporel (1) pourvu d'un conduit d'alimen-tation en sang (8) menant à l'entrée de l'unité de traitement du sang et d'un conduit de retour du sang (9) partant de la sortie de l'unité de traitement du sang, les moyens pour collecter le sang étant reliés par l'intermédiaire du premier parcours de liaison aux moyens pour stocker un gaz, et les moyens pour stocker un gaz étant reliés par l'intermédiaire du deuxième parcours de liaison aux moyens pour collecter le sang.

3. Dispositif de traitement du sang selon la revendication 1 ou 2, **caractérisé en ce que** le premier parcours de liaison présente un conduit de dérivation (21) contournant les moyens (20) pour comprimer un gaz, conduit dans lequel est disposée une vanne de dérivation (22), et le deuxième parcours de liaison présente un conduit (19) pour véhiculer un gaz, conduit qui relie les moyens (11) pour collecter le sang aux moyens (18) pour stocker un gaz, les moyens (20) pour comprimer un gaz étant disposés dans le conduit pour véhiculer un gaz.

4. Dispositif de traitement du sang selon la revendication 3, **caractérisé en ce que** le conduit (19) pour véhiculer un gaz présente un premier tronçon de conduit (19A), qui relie les moyens (11) pour collecter le sang au côté de pression des moyens (20) pour comprimer un gaz, et un deuxième tronçon de conduit (19B), qui relie le côté d'aspiration des moyens pour comprimer un gaz aux moyens (18) pour stocker un gaz, des moyens (24B) pour ventiler/purger le premier tronçon de conduit étant prévus.

5. Dispositif de traitement du sang selon l'une quelconque des revendications 1 à 4, **caractérisé en ce que** les moyens (11) pour collecter le sang sont réalisés sous la forme d'un réservoir ayant un volume prédéfini, qui est disposé dans le conduit de retour du sang (9) du circuit sanguin extracorporel (1).

6. Dispositif de traitement du sang selon l'une quelconque des revendications 1 à 5, **caractérisé en ce que** les moyens (18) pour stocker un gaz sont réalisés sous la forme d'un réservoir ayant un volume prédéfini.

7. Dispositif de traitement du sang selon l'une quelconque des revendications 1 à 6, **caractérisé en ce que** les moyens (6) pour véhiculer le sang dans le circuit sanguin extracorporel (1) sont réalisés sous la forme d'une pompe à sang qui est dispo-sée dans le conduit d'alimentation en sang (8).

8. Dispositif de traitement du sang selon l'une quelconque des revendications 1 à 7, **caractérisé en ce qu'**il est prévu des moyens (27) pour déterminer le volume sanguin $V_{Blut}$ renfermé dans les moyens (11) pour collecter le sang.

9. Dispositif de traitement du sang selon la revendication 8, **caractérisé en ce que** les moyens (27) pour déterminer le volume sanguin $V_{Blut}$ renfermé dans les moyens (11) pour collecter le sang présentent:

   des moyens (16) pour mesurer la pression dans les moyens (11) pour collecter le sang,
   des moyens (25) pour mesurer la pression dans les parcours de liaison (19, 21) entre les moyens (11) pour collecter le sang d'une part et les moyens (20) pour comprimer un gaz d'autre part, et
   des moyens (26) pour mesurer la pression dans les moyens (18) pour stocker un gaz,
   les moyens (27) pour déterminer le volume sanguin $V_{Blut}$ étant conçus de telle sorte que le volume sanguin $V_{Blut}$ est calculé sur la base de la pression mesurée dans le volume clos des moyens pour collecter le sang, dans le volume clos dans les parcours de liaison (19, 21) entre les moyens (11) pour collecter le sang d'une part et les moyens (20) pour comprimer un gaz d'autre part, et dans le volume clos des moyens pour stocker un gaz.

10. Dispositif de traitement du sang selon l'une quelconque des revendications 1 à 9, **caractérisé en ce qu'**il est prévu des moyens (27) pour passer de la phase artérielle à la phase veineuse et réciproquement, qui sont conçus de telle sorte que
dans la phase artérielle, on fait fonctionner les moyens (6) pour véhiculer le sang dans le circuit sanguin extracorporel (1), les moyens (11) pour collecter le sang étant reliés par l'intermédiaire du premier parcours de liaison (21) aux moyens (18) pour stocker un gaz, de sorte que, lors du remplissage des moyens pour collecter le sang, le gaz refoulé des moyens pour collecter le sang est transféré vers les moyens pour stocker un gaz,
dans la phase veineuse, on fait fonctionner les moyens (20) pour comprimer un gaz, les moyens (18) pour stocker un gaz étant reliés par l'intermédiaire du deuxième parcours de liaison (19) aux moyens (11) pour collecter le sang, de sorte que, afin de vider les moyens pour collecter le sang, le gaz stocké dans les moyens pour stocker un gaz est transféré par les moyens pour comprimer un gaz vers les moyens pour collecter le sang.

11. Dispositif de traitement du sang selon la revendication 10, **caractérisé en ce que** les moyens (27) pour passer de la phase artérielle à la phase veineuse et réciproquement sont réali-sés de telle sorte que, dans la phase artérielle, dans un premier intervalle de temps, les moyens (22) pour interrompre le premier parcours de liaison (21) sont actionnés de telle sorte que le premier parcours de liaison n'est pas inter-rompu, et dans un deuxième intervalle de temps, les moyens (22) pour interrompre le premier parcours de liaison (21) sont actionnés de telle sorte que le premier parcours de liaison est interrompu.

Fig. 1

Fig. 2

| Initialisierung | | | | | | | Betrieb | | |
|---|---|---|---|---|---|---|---|---|---|
| 1 | 2 | 3 | 4 | 5 | 6 | 7 | 1. venöse Phase | arterielle Phase | venöse Phase |

**Füllstand**
oberer Umschaltpunkt

Leveldetektor

unterer Umschaltpunkt

Zeit

**Drücke**

gewünschter
Rückgabedruck

Leitungsdruck

Kammerdruck

Umgebungsdruck

Speicherdruck

Zeit

**Gesamt-
Luftmasse
im System**

Zeit

**Druck**

gewünschter
Rückgabedruck

| arterielle Phase | venöse Phase |
|---|---|

Leitungsdruck

SN-Kammerdruck

Speicherdruck

| Bypassventil geöffnet | Bypassventil geschlossen |
|---|---|
| Kompressor steht, Blutpumpe fördert | Kompressor regelt, Blutpumpe steht |
| venöse Klemme geschlossen | venöse Klemme geöffnet |

**Zeit**

## Fig. 3

EP 2 152 335 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0472480 A
- DE 102005001779 A1

- EP 0405094 A2